Europäisches Patentamt

European Patent Office

Office européen des brevets

(11) Publication number: **0 401 832**
**A2**

## (12) EUROPEAN PATENT APPLICATION

(21) Application number: 90110823.3

(51) Int. Cl.⁵: **A61K 9/48, A61J 3/07**

(22) Date of filing: 07.06.90

(30) Priority: 08.06.89 JP 145626/89

(43) Date of publication of application:
**12.12.90 Bulletin 90/50**

(84) Designated Contracting States:
**DE FR GB IT**

(71) Applicant: **Shin-Etsu Chemical Co., Ltd.**
**6-1, Ohtemachi 2-chome**
**Chiyoda-ku Tokyo 100(JP)**

(72) Inventor: **Araume, Kiyoshi, Shin-Etsu**
**Chemical Co., Ltd.**
**Synthetic Tech. Lab. 8-1, Oaza**
**Nishi-Fukushima**
**Kubiki-mura, Nakakubiki-gun, Nigata 942(JP)**
Inventor: **Muto, Hiroaki, Shin-Etsu Chemical**
**Co., Ltd.**
**Synthetic Tech. Lab. 8-1, Oaza**
**Nishi-Fukushima**
**Kubiki-mura, Nakakubiki-gun, Nigata 942(JP)**
Inventor: **Nishiyama, Yuschi, Shin-Etsu**
**Chemical Co., Ltd.**
**Synthetic Tech. Lab. 8-1, Oaza**
**Nishi-Fukushima**
**Kubiki-mura, Nakakubiki-gun, Nigata 942(JP)**
Inventor: **Chiba, Tohru, Shin-Etsu Chemical**
**Co., Ltd.**
**Synthetic Tech. Lab. 8-1, Oaza**
**Nishi-Fukushima**
**Kubiki-mura, Nakakubiki-gun, Nigata 942(JP)**

(74) Representative: **Bühling, Gerhard, Dipl.-Chem.**
**et al**
**Patentanwaltsbüro Tiedtke-Bühling-Kinne**
**Grupe-Pellmann-Grams-Struif Bavariaring 4**
**D-8000 München 2(DE)**

(54) Method for preparing hard capsules for medicaments.

(57) Method for preparing hard capsules for medicaments is herein disclosed and the method comprises the steps of immersing pins each having and external shape corresponding to that for a body or a cap of the capsule in an aqueous solution of hydroxypropylmethyl cellulose having structural viscosity as one of its flow characteristics, pulling up the pins whose surfaces are covered with the aqueous solution, drying the solution to form molded products of the bodies or the caps, releasing the molded products from the pins, cutting the resulting products into pieces having a desired length and then fitting the resulting bodies into the resulting caps. The method makes it possible to easily provide hard capsules which have uniform thickness of the wall thereof. Thus, the resulting capsules are not broken during filling the medicaments therein.

## Method for Preparing Hard Capsules for Medicaments

The present invention relates to a method for preparing hard capsules for packing with medicaments from a starting material composed of hydroxypropylmethyl cellulose which is a water-soluble derivative of cellulose.

A hard capsule for medicaments in general comprises a body in which a medicament is filled and a cap into which the body is fitted. There has widely been used gelatin as a principal ingredient therefor.

U. S. Patent No. 1,787,777 granted to Colton discloses a method for preparing a capsule from gelatin. In this method, pins are employed, each having an external shape corresponding to the body portion of the capsule or the cap portion thereof. More specifically, the pins are immersed into a hot aqueous solution of gelatin and then pulled up therefrom, whereby the surfaces of the pins are coated or covered with the gelatin aqueous solution. As the layer of the aqueous gelatin solution on the pin is cooled down to room temperature, it gels at a predetermined thickness. Then theses are dried by cold air having a temperature of not more than the gelatin temperature of the aqueous gelatin solution, the dried gel is released from the pin and cut into pieces having a predetermined length to thus give a cap and a body.

Gelatin is in general fragile and hence liable to be broken down upon drying. For this reason, the water content in gelatin should be controlled to a desired level or higher in order to handle such a capsule made from gelatin without any troubles and to ensure its functions as a container for medicaments. However, such humidity of gelatin possibly causes deterioration of the medicaments contained herein and a reaction between the medicaments with gelatin to thus often impair the potency thereof. In addition, gelatin is liable to cause contamination with microorganisms in the presence of humidity.

As substitutes for gelatin having such problems, there have been used, for instance, water-soluble cellulose derivatives, starches, starch derivatives, polyvinyl alcohols and sodium alginate as starting materials for preparing hard capsules for packing with various medicaments and they have already been put in practical use.

Further U. S. Patent No. 2,526,683 granted to Murphy discloses a method for preparing capsules by immersing, in an aqueous solution of methyl cellulose, pins which have been heated to a temperature of not less than the gelatin temperature of the aqueous solution in advance, then pulling up the pins and drying the layer of the solution on the pins to give a capsules.

U. S. Patent No. 3,493,407 granted to Greminger et al. discloses a method for preparing such capsules in which a hydroxyalkylalkyl cellulose is used as a starting material and a mixture of water and an alcohol or a mixture of an alcohol and a chlorinated hydrocarbon or an aromatic hydrocarbon is employed as a solvent for preparing a solution of the cellulose derivative, the hydroxyalkylalkyl cellulose comprising 4 to 15% of hydroxyalkoxy groups having 2 to 4 carbon atoms and 18 to 32% of alkoxy groups having 1 to 2 carbon atoms; and a viscosity of 2 to 20 cps as determined on 2% by weight aqueous solution at 20 $^{\circ}$ C .

U. S. Patent No. 3,617,588 granted to Langman discloses a method for preparing capsules from a hydroxyalkylalkyl cellulose which has hydroxyalkoxy groups having 2 to 4 carbon atoms at a degree of substitution ranging from 0.07 to 1.0 and alkyl groups at a degree of substitution ranging from 0.6 to 2.0 and which has a viscosity of 2 to 20 cps as determined on a 2% by weight aqueous solution at 20 $^{\circ}$ C . More specific- ally, this patent discloses a method for preparing a capsule, which comprises the steps of immersing pins having a conductive surface in an aqueous solution of the cellulose having a concentration of at least 20% by weight, pulling up the pins and, immediately thereafter, heating the surface of the pins by an electromagnetic induction coil to thus cause gelatin and to dry the resulting gel.

U. S. Patent No. 4,001,211 granted to Sarkar discloses an improved methyl cellulose ether composition from which capsules for packing with medicaments are prepared according to method which comprises the step of immersing preheated pins in an aqueous solution of the composition. This composition has methoxy groups at a degree of substitution ranging from 1.5 to 2.0 and hydroxyalkoxy groups having 2 to 3 carbon atoms at a degree of substitution ranging from 0.1 to 0.4 and has a viscosity of 2 to 10 cps as determined on a 2% by weight aqueous solution at 20 $^{\circ}$ C ; a thermal gel point ranging from 50 to 80 $^{\circ}$ C ; and a viscosity ranging from about 1,000 to 10,000 cps as determined on a 15 to 30% by weight aqueous solution. The composition is further characterized by having, as a 15 to 30% by weight aqueous solution, essentially Newtonian fluid properties as defined by a power law coefficient, n, of 0.9 to 1.0 at shear rates of between 0.1 to 10 sec$^{-1}$ and a 50 sec gel yield strength of at least 150 dynes/cm$^2$ at 65 $^{\circ}$ C .

European Patent Application Publication No. 246,693 dated 25. 11. 87 in Bulletin 87/48 by Shin-Etsu Chemical Co., Ltd.; corresponding to Japanese Patent Un-examined Published Application Nos. 61-100519 and 62-266060) discloses hard capsules for medicaments use obtained from a mixture of a cellulose ether substituted with alkyl groups and/or hydroxyalkyl groups and a polyvinyl alcohol as a principal ingredient,

2

According to this patent application, hard capsules can be obtained by dipping pins in an aqueous solution of hydroxypropylmethyl cellulose, pulling up the pins, dipping the pins adhered the aqueous solution in hot water maintained at a temperature of not less than the gelatin temperature of the aqueous solution and then drying it.

Among the foregoing techniques, the characteristic properties of the aqueous solution of hydroxypropylmethyl cellulose are described in the Sarkar's patent, but the latter patent simply discloses that the aqueous solution has flow properties as an essentially Newtonian fluid and it does not disclose the variation, with time, in the flow properties of the solution at all.

Uniformity in thickness is a very important factor for capsules for packing with medicaments. The thickness of the capsule body and the wall of the cap must be uniform as much as possible. For instance, if a part of the wall of the cap and the body is thick, they cannot properly be inserted into holes for supporting the same provided to a capsule filler or further normal engagement of the cap with the body cannot be ensured and hence they are possibly in gear with one another to thus be finally broken.

Unevenness in the thickness of the capsule wall is resulted from the flowing down of a solution of ingredients adhered to the surface of pins after the pins are immersed in the solution and then pulled up during the formation of the capsules. In the case of gelatin, pins maintained at room temperature in the order of about 25°C are immersed in a solution heated to about 40°C, pulled up and thus the solution adhered to the surfaces of the pins is cooled to give a gel layer. For this reason, the solution only slightly flows down and, therefore, the thickness of the wall of the resulting capsule becomes uniform. On the other hand, the aqueous hydroxypropylmethyl cellulose solution is heated to cause gelatin and thus pins heated in advance are in general immersed in the solution to form capsules thereof. Upon immersing the pins in the solution, the temperature of the solution is raised in the vicinity of the surface of the pins and thus the gelatin thereof starts. However, the flowing down of the solution is liable to cause at the initial stage of drying after the pins are pulled up. This often leads to the formation of capsules having a non-uniform thickness. In the worst case, the portions at which the solution flows down wrinkle. This is because, the water in the coated solution is evaporated during initial drying stage, the temperature of the pins drops due to the heat of evaporation and hence the gelatin of the solution which proceeds during the immersion and pulling up processes is interrupted.

As has been explained hereinbefore, when capsules having a uniform wall-thickness are prepared from an aqueous solution of hydroxypropylmethyl cellulose, it is required to minimize the flowing down of the solution adhered to the surface of pins observed during the initial stage of drying. One of the solutions for this problem is to increase the viscosity of the aqueous solution. However, a highly viscous solution must be stirred for a long time period for removing air bubbles present in the solution and the rate of pulling up of the pins must be reduced to adhere a desired amount of the solution onto the pin surface. This leads to the reduction in the producibility of the capsules. Another means for solving the problem is to increase the drying temperature after pulling up the pins and to dry the coated solution with hot air. However, the coated solution is non-uniformly dried and thus the resulting capsules wrinkle. Thus, it is difficult to prepare capsules having a uniform wall-thickness from and aqueous solution of hydroxypropylmethyl cellulose.

Generally, an object of the present invention is to eliminate the drawbacks associated with the foregoing conventional techniques and more specifically to provide hard capsules for packing with medicines obtained from hydroxypropylmethyl cellulose as a starting material and having a uniform wall-thickness.

The inventors of this invention have conducted various studies on the flow properties of an aqueous solution of hydroxypropylmethyl cellulose to thus solve the foregoing problems, have found that the solution must have a structural viscosity in order to prevent the flowing down of the solution adhered to the surface of pins observed at the initial stage of drying after the pins are immersed in the solution and then pulled up during the preparation of capsules and have completed the present invention on the basis of this finding.

In the method for preparing hard capsules for packing with medicaments according to the present invention, there is employed and aqueous solution of hydroxypropylmethyl cellulose having a structural viscosity as flow properties. The aqueous solution adhered to the surface of the pins pulled up is immediately thickened due to its structural viscosity. Therefore, the solution very slightly flows down even if the temperature of the pin is dropped due to the heat of evaporation of the water present in the solution. Thus, according to the method of this invention, capsules for medicaments having a uniform wall-thickness can effectively be prepared.

The foregoing object of the present invention can effectively be achieved by providing a method for preparing hard capsules for packing with medicaments which comprises the steps of immersing pins each having an external shape corresponding to that for a body or a cap for the capsule in an aqueous solution of hydroxypropylmethyl cellulose having structural viscosity as one of its flow characteristics, pulling up the pins whose surfaces are covered with the aqueous solution, drying the solution to form molded products,

3

releasing the molded products from the pins, cutting the resulting products into pieces having a desired length and then fitting the resulting capsule bodies into the resulting capsule caps.

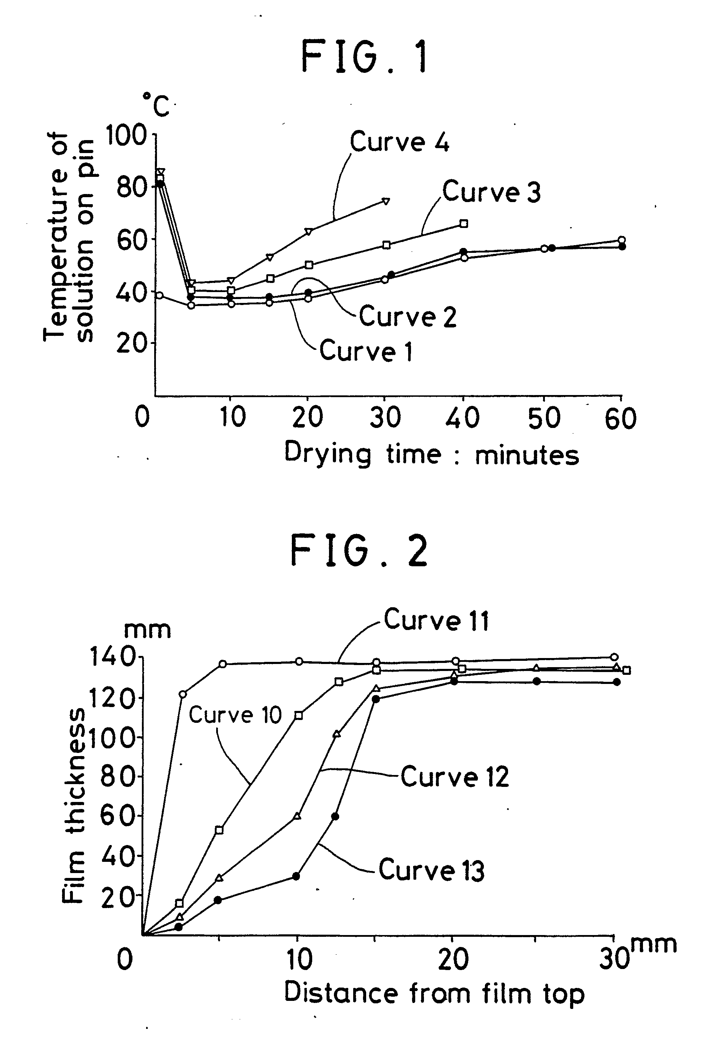

Fig. 1 is a diagram for illustrating the relation between the drying time and the temperature at which the solution is applied to the surface of pins; and Fig. 2 is a diagram for illustrating the relation between the distance from the top of the film and the thickness of the film at the corresponding distance in the evaluation test of flowing down of the solution.

The method for preparing capsules for containing medicaments according to the present invention will hereinafter be explained in more detail.

The foregoing solution used for forming capsules is aqueous solution of hydroxypropylmethyl cellulose having structural viscosity. A viscosity of aqueous solution of 15 to 25% by weight hydroxypropyl- methyl cellulose ranges from 3,000 to 15,000 cps at 40 °C . The aqueous solution preferably has a ratio of the viscosity determined after allowing to stand for 30 minutes while maintaining the temperature at 40 °C to that determined immediately after being sufficiently stirred at a shear rate of 1.5 sec$^{-1}$ of not less than 1.3 : 1.0. In order to make the application of the solution more easier and to avoid air bubbles from coming into the aqueous solution, the viscosity determined at 40 °C more preferably ranges from 3,000 to 10,000 cps. The foregoing ratio as a requirement for viscosity preferably ranges from 1.3 : 1.0 to 10 : 1.0. This is because if the ratio is less than 1.3 : 1.0, the aqueous solution adhered to the surface of a pin is liable to cause flowing down during the formation of the capsules and thus the wall-thickness of the resulting capsules becomes uneven. On the other hand, if the ratio exceeds the upper limit 10 : 1.0, since the viscosity of the solution may vary by the growth of the structural viscosity thereof in short time, it cause to difficult to handle the solution.

Hydroxypropylmethyl cellulose having a viscosity ranging from 3.0 to 15.0 cps as determined on a 2% aqueous solution thereof at 20 °C is employed in the present invention. The aqueous solution having a viscosity measured at 40 °C ranging from 3,000 to 15,000 cps can be obtained by dissolving the hydroxypropylmethyl cellulose, which satisfies the foregoing requirement for viscosity, in water so that the concentration thereof falls within the range of from 15 to 25% by weight.

The hydroxypropylmethyl cellulose preferably has a degree of substitution with methoxy groups per glucose residue ranging from 1.0 to 1.5. This is because if it is less than 1.0, the solubility of the resulting capsule in water becomes insufficient. On the other hand, it is more than 1.5, the aqueous solution severely flows down during the formation of the capsules and hence the wall-thickness of the resultant capsule becomes non-uniform.

In addition, the hydroxypropylmethyl cellulose preferably has a degree of substitution with hydroxypropoxy groups per glucose residue ranging from 0.1 to 0.3. This is because if it is less than 0.1, the solubility of the resulting capsule in water becomes insufficient. On the other hand, it is more than 0.3, the aqueous solution severely flows down during the formation of the capsules and hence the wall-thickness of the resultant capsule becomes non-uniform.

The aqueous solution may further comprise a water-soluble food dyestuff as a coloring agent and titanium dioxide as a masking agent. Moreover, it is also possible to incorporate, into the aqueous solution, polyethylene glycol as a plasticizer or a surfactant for the purposes of improving the flexibility of the resulting capsule wall.

The aqueous solution must be prepared so as to control the number of air bubbles present therein as low as possible. For this reason, the hydroxypropylmethyl cellulose is added to hot water having a temperature of not less than 80 °C and the temperature is held at this level for about 1 to 2 hours to make the particles of the cellulose sufficiently compatible with hot water. After thus removing air bubbles present between the particles, the solution is allowed to stand to thus cool the solution while gently stirring it whereby a uniform solution is obtained.

When capsules are prepared, the aqueous solution is maintained at a constant temperature ranging from 20 to 40 °C . The temperature of the pin is controlled so that it falls within the range of from room temperature to 70 °C . After the pin is pulled up from the solution, the pin which is covered with a layer of the solution can be, for instance, immersed in a hot water bath or heated with a heater to temporarily immobilize the layer by the gelatin of the coated layer for the purpose of preventing the layer from flowing down before the pin is introduced into a dryer. The drying thereof is preferably performed in a dryer free of air circulation and the drying temperature desirably ranges from 60 to 80 °C .

The present invention will hereunder be further described in more detail with reference to the following non- limitative working Examples and the effects practically achieved by the present invention will be discussed in detail in comparison with Comparative Examples given below.

Before capsules were prepared according to the method of this invention, the inventors examined temperature change of the layer of solution applied to the surface of a pin observed during the drying process in the method for preparing the capsules. The measurement of the temperature change was performed by a pin welded a thermocouple. The preheated or not preheated pins were immersed in an aqueous solution of hydroxypropylmethyl cellulose, pulled up therefrom, immersed in hot water or not immersed and transferred to a dryer to dry the layer of the solution adhered to the surface of the pin. The temperature change was detected by the thermocouple connected to the pin in constant intervals to thus give the relation between the drying time and the temperature of the coated solution.

The resulting relation between the time and the temperature was plotted on Fig. 1. In Fig. 1, the curve 1 corresponds to the temperature change observed under the following conditions: the temperature for preheating the pin was 60°C ; the temperature of the solution in which the pin was immersed was 40 °C ; the pin was not immersed in hot water bath; and the drying temperature (the temperature of the dryer) was 60°C . The curve 2 correspond to the temperature change in the layer of the solution adhered to the pin which was observed under the following conditions: the pin was immersed in the solution without preheating (i. e., its temperature was room temperature in the order of about 25 °C ); the temperature of the solution in which it was immersed was 20°C ; it was immersed in hot water of 90°C for 20 seconds before drying the layer of the solution in the dryer maintained at a temperature of 60 °C . The curve 3 corresponds to the temperature change of the layer of the solution observed under the same conditions as those for curve 2 except that the temperature of the dryer was changed to 70 °C . The curve 4 corresponds to the temperature change of the layer of the solution observed under the same conditions as those for curve 2 except that the temperature of the dryer was changed to 80°C . As seen from the results plotted on Fig. 1 as the curves 1 to 4, the temperature of the coated solution observed during the initial stage of the drying (i. e., observed during the drying time ranging from 5 to 15 minutes) always falls within the range of from 35 to 45 °C irrespective of whether the pin was preheated or not; the drying time; and whether it was treated with hot water or not.

Then, the following experiments were performed using samples having different degrees of substitution in order to search for preferable hydroxypropylmethyl cellulose of which structural viscosity of aqueous solution immediately grew at 35 to 45°C and of which aqueous solution adhered to pins scarcely flowed down along the pins after the pins were pulled up from the solution. As listed in Table 1 give below, three kinds of hydroxypropylmethyl cellulose differing in the degrees of substitution with methoxy groups and that with hydroxypropoxy groups were dissolved in water to form Samples 1 to 3. 500 g each of these aqueous solutions (Samples 1 to 3) was added to a 500 ml beaker having a diameter of 85 mm, the beaker was warmed in a water bath maintained at 40°C and was stirred for 2 hours at about 200 rpm with a frame-like stirring blade having a width of 4 cm and height of 5.5 cm. Immediately after the interruption of the stirring, the viscosity A of each of Samples was determined with a Brookfield viscometer (BL viscometer; No. 3 rotor; available from TOKYO KEIKI CO.,LTD.) at 6 rpm, a shear rate of 1.48 sec$^{-1}$. Thereafter, the viscosity B of each of solutions was likewise determined after allowing to stand at 40 °C for 30minutes. The results thus obtained are summarized in Table 1. The viscosity of a 2% aqueous solution containing either of these three kinds of hydroxypropyl methyl cellulose determined at a temperature of 20°C are also listed in Table 1.

Table 1

| Sample No. | 1 | 2 | 3 |
|---|---|---|---|
| Degree of substitution with methoxy groups | 1.4 | 1.8 | 1.9 |
| Degree of substitution with hydroxypropoxy groups | 0.20 | 0.23 | 0.25 |
| Viscosity; cps (2% aqueous solution, at 20 °C ) | 6.1 | 6.3 | 6.5 |
| Concentration of the solution (%) | 17.1 | 17.6 | 17.5 |
| Viscosity A (cps) determined immediately after the interruption of stirring | 4600 | 4750 | 4300 |
| Viscosity B (cps) determined after allowing to stand for 30 minutes | 6400 | 5750 | 4950 |
| Ratio of viscosity B to viscosity A; B/A X 100 (%) | 139 | 121 | 115 |

It is clear, from the results shown in Table 1, that Sample 1 most favorably causes the desired structural viscosity.

In general, the viscosity of non-Newtonian solutions increases as the shear rate increases, while if the shear rate is too high, the structure of the non-Newtonian solutions are possibly broken and, therefore, the true structural viscosity thereof cannot often be obtained. For this reason, the structural viscosity must be determined at a shear rate as low as possible, but if it is too low, the precision of the resulting viscosity value becomes low. Thus measurement of viscosity ranging from 3,000 to 15,000 cps which is suitable for the immersion and coating processes in the method of the present invention is preferably performed at a shear rate ranging from 1.0 to 2.0 sec⁻¹ and approximately constant viscosity can be obtained at a shear rate falling within the range defined above.

Then, the inventors of this invention evaluated the degree of flowing down of the solution observed during the initial drying stage using the foregoing aqueous solutions of Samples 1 to 3.

In an atmosphere maintained at 40 °C, each aqueous solution was poured on the surface of a glass plate having a size of 10 x 10 cm, spread thereon with an applicator so that the thickness thereof was equal to 1 mm and immediately after the spreading, the glass plate was vertically stood on a fixed stand and allowed to stand for one minute. The glass plate was then horizontally placed in a dryer to dry and thus form a film and the relation between the distance from the top of the film and the thickness of the film observed at the corresponding distance was determined. This relation observed on Sample 1 was likewise determined in an atmosphere maintained at 20 °C . The results thus obtained are plotted in Fig. 2. The curve 11 in Fig. 2 shows the relation between the distance from the top of the film and the thickness of the film at the corresponding distance determined on the film obtained from the aqueous solution of Sample 1 in the atmosphere of 40°C . The curve 12 shows the same relation observed on the film obtained from the aqueous solution of Sample 2 in the atmosphere of 40 °C . The curve 13 shows the same relation observed on the film obtained from the aqueous solution of Sample 3 in the atmosphere maintained at 40°C . The curve 10 shows the same relation observed on the film obtained from the aqueous solution of Sample 1 in the atmosphere maintained at 20°C .

It was found, from the results shown in Fig. 2, that the lowest flowing down of the solution was observed on the aqueous solution of Sample 1.

According to the method of the present invention, capsules were practically prepared and the quality thereof was evaluated. These Examples will be detailed below.

Example 1

In this Example, the aqueous solution (Sample 1) listed in Table was employed as a starting solution. To the surface of a pin having the external shape corresponding to the capsules to be formed, there was applied a thin layer of liquid paraffin as a releasing agent and it was heated to 60°C in advance. The pins were immersed in the aqueous solution of Sample 1 maintained at 40 °C and were pulled up from the solution over 30 seconds. The pins were then dried in a dryer free of air circulation maintained at 65°C for 50 minutes, then the molded products were released from the pins and cut into pieces having a predetermined length to give bodies of capsules.

Comparative Example 1

Bodies of capsules were prepared according to the same procedures used in Example 1 except that the aqueous solution of Sample 2 listed in Table 1 was substituted for the aqueous solution of Sample 1 used in Example 1.

Comparative Example 2

Bodies of capsules were prepared according to the same procedures used in Example 1 except that the aqueous solution of Sample 3 listed in Table 1 was substituted for the aqueous solution of Sample 1 used in Example 1.

Example 2

There was prepared, as a starting aqueous solution, a 17.0% by weight aqueous solution of hydroxypropylmethyl cellulose whose degree of substitution with methoxy groups (DS) of 1.2 and that with hydroxypropoxy groups (MS) of 0.22. The viscosity of this solution determined at a temperature of 20°C was 6.2 cps. In addition, this aqueous solution had a viscosity determined at 40 °C immediately after stirring of 4650 cps and the viscosity thereof determined at 40°C after stirring followed by allowing to stand for 30 minutes was increased by 65% that of the former.

This aqueous solution was maintained at a temperature of 20°C, pins to which a small amount of liquid paraffin as a releasing agent had been applied and preheated to 25°C were immersed in the aqueous solution and were pulled up from the solution over 30 seconds. Then the pins were immersed in hot water maintained at 98°C for 15 seconds and pulled up therefrom. The pins were dried in a dryer free of air circulation maintained at 65°C for 50 minutes, then the molded products thus formed were released from the pins and cut into pieces having a predetermined length to thus give bodies of capsules.

Comparative Example 3

The same procedures as those used in Example 2 were repeated to obtain bodies of capsules except that the aqueous solution of Sample 2 listed in Table 1 was substituted for the starting aqueous solution used in Example 2.

The wall portion of each of the capsule bodies thus obtained in Examples and Comparative Examples was folded in two, then the thickness thereof in the longitudinal direction was determined with a dial gauge and 1/2 of the resulting value was defined to be the thickness of the wall. In addition, the difference between the maximum thickness and the minimum thickness of the film in the circumferential direction of the wall determined at the central portion of the wall with a pipe gauge. The bodies obtained in Comparative Examples 1 and 2 wrinkled on the wall from the upper portion even to the central portion thereof.

In Table 2 given below, there are summarized the thickness of the wall at upper, middle and lower portions thereof and the difference between the maximum thickness and the minimum thickness of the film at the central portion of the wall observed on each Samples.

Table 2

|  | Ex. 1 | Comp. Ex. 1 | Comp. Ex. 2 | Ex. 2 | Comp. Ex. 3 |
|---|---|---|---|---|---|
| Wall Thickness ($\mu$m) | | | | | |
| Upper portion | 106 | 165 | 245 | 102 | 125 |
| Middle portion | 80 | 280 | 350 | 95 | 104 |
| Lower portion | 78 | 94 | 110 | 98 | 86 |
| Difference in Film Thickness ($\mu$m) | 36 | 85 | 130 | 23 | 45 |

As has been described above in detail, the method of the present invention makes it possible to easily provide capsules for packing with various medicaments, which have uniform thickness of the wall. Therefore, the capsules are never broken during filling of the medicaments. Method for preparing hard capsules for medicaments is herein disclosed and the method comprises the steps of immersing pins each having and external shape corresponding to that for a body or a cap of the capsule in an aqueous solution of hydroxypropylmethyl cellulose having structural viscosity as one of its flow characteristics, pulling up the pins whose surfaces are covered with the aqueous solution, drying the solution to form molded products of the bodies or the caps, releasing the molded products from the pins, cutting the resulting products into pieces having a desired length and then fitting the resulting bodies into the resulting caps. The method makes it possible to easily provide hard capsules which have uniform thickness of the wall thereof. Thus, the resulting capsules are not broken during filling the medicaments therein.

**Claims**

1. A method for preparing hard capsules for medicaments comprising the steps of immersing pins each having an external shape corresponding to that for a body or a cap for the capsule in an aqueous solution of hydroxypropylmethyl cellulose having structural viscosity as one of its flow characteristics, pulling up the pins whose surface is covered with the aqueous solution, drying the solution to form molded products of the body or the cap, releasing the molded products from the pins, cutting the resulting products into pieces having a desired length and then fitting the resulting capsule bodies into the resulting capsule caps.

2. The method for preparing hard capsules for medicaments as set forth in claim 1 wherein the aqueous solution of hydroxypropylmethyl cellulose has a concentration ranging from 15 to 25% by weight, a viscosity determined at 40°C ranges from 3,000 to 15,000 cps and at least 1.3 : 1.0 of a ratio of the viscosity determined after stirring and then allowing to stand the solution for 30 minutes while maintaining the temperature at 40°C to that determined immediately after sufficiently stirring the solution at 1.5 sec$^{-1}$ of a shear rate.

3. The method for preparing hard capsules for medicaments as set forth in claim 1 wherein the aqueous solution of hydroxypropylmethyl cellulose has a concentration ranging from 15 to 25% by weight, a viscosity determined at 40°C ranges from 3,000 to 10,000 cps and 1.3 : 1.0 to 10 : 1.0 of a ratio of the viscosity determined after stirring and then allowing to stand the solution for 30 minutes while maintaining the temperature at 40°C to that determined immediately after sufficiently stirring the solution at 1.5 sec$^{-1}$ of a shear rate.

4. The method for preparing hard capsules for medicaments as set forth in claim 1 wherein the hydroxypropylmethyl cellulose has a viscosity ranging from 3.0 to 15.0 cps as determined on a 2% aqueous solution thereof at 20°C.

5. The method for preparing hard capsules for medicaments as set forth in claim 1 wherein the hydroxypropylmethyl cellulose has a degree of substitution with methoxy groups per glucose residue ranging from 1.0 to 1.5.

6. The method for preparing hard capsules for medicaments as set forth in claim 1 wherein the hydroxypropylmethyl cellulose has a degree of substitution with hydroxypropoxy groups per glucose residue ranging from 0.1 to 0.3.

7. The method for preparing hard capsules for medicaments as set forth in claim 1 wherein the hydroxypropylmethyl cellulose has a degree of substitution with methoxy groups per glucose residue ranging from 1.0 to 1.5 and a degree of substitution with hydroxypropoxy groups per glucose residue ranging from 0.1 to 0.3.

8. The method for preparing hard capsules for medicaments as set forth in claim 1 wherein a coloring agent is incorporated into the aqueous solution.

9. The method for preparing hard capsules for medicaments as set forth in claim 8 wherein a water-soluble food dyestuff, red oxide or a natural dyestuff, as the coloring agent, is incorporated into the aqueous solution.

10. The method for preparing hard capsules for medicaments as set forth in claim 1 wherein titanium oxide is incorporated into the aqueous solution.

11. The method for preparing hard capsules for medicaments as set forth in claim 1 wherein a plasticizer is incorporated into the aqueous solution.

12. The method for preparing hard capsules for medicaments as set forth in claim 1 wherein the pins are immersed in the aqueous solution while the aqueous solution is maintained at a temperature ranging from 20 to 40°C.

13. The method for preparing hard capsules for medicaments as set forth in claim 1 wherein the pins are immersed in the aqueous solution after they are preheated to a temperature ranging from 20 to 70°C.

14. The method for preparing hard capsules for medicaments as set forth in claim 1 wherein the pins are pulled up from the aqueous solution, then immersed in a hot water bath to caused gelatin and then dried.

15. The method for preparing hard capsules for medicaments as set forth in claim 1 wherein the drying is performed in a dryer free of air circulation and maintained at a temperature ranging from 60 to 80°C.

# FIG. 1

# FIG. 2